Europäisches Patentamt

European Patent Office

Office européen des brevets

(19)

(11) **EP 1 466 636 A1**

(12) **EUROPEAN PATENT APPLICATION**
published in accordance with Art. 158(3) EPC

(43) Date of publication:
**13.10.2004 Bulletin 2004/42**

(51) Int Cl.7: **A61M 1/36**, B01J 20/26

(21) Application number: **02805884.0**

(86) International application number:
**PCT/JP2002/013207**

(22) Date of filing: **18.12.2002**

(87) International publication number:
**WO 2003/055545 (10.07.2003 Gazette 2003/28)**

(84) Designated Contracting States:
**DE FR GB IT**

(30) Priority: **25.12.2001  JP 2001391136**

(71) Applicant: **KANEKA CORPORATION
Osaka-shi, Osaka 530-8288 (JP)**

(72) Inventors:
• **HIRAI, Fumiyasu, c/o KANEKA CORPORATION
Settsu-shi, Osaka 566-0072 (JP)**

• **FUJIMOTO, Tamiji, c/o KANEKA CORPORATION
Settsu-shi, Osaka 566-0072 (JP)**
• **FURUYOSHI, Shigeo,
c/o KANEKA CORPORATION
Settsu-shi, Osaka 566-0072 (JP)**

(74) Representative: **HOFFMANN - EITLE
Patent- und Rechtsanwälte
Arabellastrasse 4
81925 München (DE)**

(54) **ADSORBENT FOR CYTOKINE, METHOD OF ADSORPTIVE REMOVAL, AND APPARATUS FOR ADSORPTIVE REMOVAL**

(57)    The present invention provides an adsorbent for efficiently adsorbing and removing cytokine in liquid and a method for removing cytokine in liquid by the adsorbent. Also, the present invention provides an adsorber for removing cytokine in liquid. Specifically, an adsorbent for cytokine is obtained by immobilizing a compound having a log P value of at least 2.50 (P is the partition coefficient in an octanol-water system) on a water-insoluble carrier. By contacting a liquid containing cytokine with the adsorbent for cytokine, the cytokine in the liquid can be effectively adsorbed and removed. Furthermore, cytokine can be efficiently adsorbed and removed by an adsorber comprising a container having an inlet and an outlet for liquid and a means for preventing the adsorbent from flowing out of the container, which is filled with the adsorbent.

FIG. 1

## Description

TECHNICAL FIELD

The present invention relates to an adsorbent for adsorbing and removing at least one cytokine selected from the group consisting of interleukin-4 (hereinafter referred to as IL-4), interleukin-10 (hereinafter referred to as IL-10) and interleukin-13 (hereinafter referred to as IL-13) from body fluids, a method for adsorbing and removing the cytokine with the adsorbent and an adsorber for the cytokine.

BACKGROUND ART

Immunocytes produce various active substances when causing immune response. Some are proteinous substances called cytokine, and play a very important role as biophylatic factors, which are closely involved in immune inflammatory reactions specific or nonspecific to various antigens. Although indispensable for maintenance of homeostasis of a living body, cytokines are excessively produced in conditions such as inflammation and are involved in the pathogenesis and prolongation of inflammation.

IL-4 is a substance which was reported to be a B cell activating factor in 1982 by Howard and Paul, and also by Vitetta et al. Thereafter, this substance was found to act on cells other than B cells and given the name of IL-4. IL-4 is a glycoprotein having a molecular weight of 20 kDa. T cells and mast cells are known as IL-4 producing cells. As the functions of IL-4, there are many functions such as the activation of B cells, induction of differentiation and growth of B cells, induction of growth of T cells and differentiation of T cells to Th2 cells, induction of thymocyte growth, induction of NK cell- and LAK cell-activation, eosinophilic anti-tumor activity, macrophage anti-inflammatory activity, induction of mast cell growth, enhancement of expressions of fibroblast/vascular endotherial cell adhesion molecules and enhancement of hematopoietic cell colony formation.

IL-10 is a substance which was identified as a cytokine production inhibitory factor in 1989 by Mosmann et al. Initially, the substance was called a cytokine production inhibitory factor but was renamed as IL-10 because the substance was found to have various other activities as well. IL-10 is a homodimer-type glycoprotein having a molecular weight of 35 to 40 kDa. As IL-10 producing cells, known are Th0/Th2 cells, activated T cells, monocytes/macrophages, activated B cells, melanocytes, and the like. The functions of IL-10 include suppression of IFN-$\gamma$ production in T cells, induction of T cell growth, induction of B cell growth, enhancement of monocyte ADCC activity, and suppression of monokine production in monocytes/macrophages.

IL-13 is a substance which was cloned in 1993 by Minty et al. in France and Brown et al. in USA. When IL-13 gene is introduced into COS cells, the gene is expressed as a glycoprotein having a molecular weight of 17 kDa. Activated T cells are known as IL-13 producing cells. As functions of IL-13, there are suppression of inflammatory cytokine production in LPS-stimulated monocytes, prolongation of the survival time of monocytes and induction of expression of CD23 and MHC Class II on the surface of monocytes and B cells.

These cytokines, IL-4, IL-10 and IL-13, are called anti-inflammatory cytokine, and all have functions of anti-inflammatory activity in common, such as macrophage anti-inflammatory activity, suppression of monokine production in monocytes/macrophages and suppression of inflammatory cytokine production in LPS-stimulated monocytes.

Recently, there are reports that in the condition of excessive biological reaction caused by invasion, which is classified under the new concept of systemic inflammatory response syndrome (SIRS), protein called inflammatory cytokines such as interleukin-1 (hereinafter referred to as IL-1), interleukin-6 (hereinafter referred to as IL-6), interleukin-8 (hereinafter referred to as IL-8) and tumor necrosis factor-$\alpha$ (hereinafter referred to as TNF-$\alpha$) are overproduced. The activities thereof have been reported to mainly cause systemic inflammatory reaction, followed by not only tissue disorders and multi-organ insufficiency, but also death.

Anti-inflammatory cytokines have been considered to suppress the action of these inflammatory cytokines. However, recently, in conditions which are included in the concept of compensatory anti-inflammatory response syndrome (CARS), an excessive amount of anti-inflammatory cytokines is produced and as a result, suppression of immune reaction occurs, whereby a condition of easily being infected is caused. Therefore, in the case of infection such as septicemia, organopathy also occurs.

In any case, cytokines should work locally and increase in concentration of cytokines in blood is undesirable for the human body. Therefore, a method for removing these cytokines from blood is desired.

An adsorbent for removing IL-1, IL-6, IL-8 and TNF-$\alpha$ which are called inflammatory cytokine has already been disclosed (Japanese Unexamined Patent Publication Nos. 257115/1996 and 257398/1996). These publications disclose that these inflammatory cytokines can be adsorbed and removed with an adsorbent comprising a compound having a log P value of at least 2.50, which is immobilized on a water-insoluble carrier.

In the present invention, anti-inflammatory cytokines, of which the physiological activities with respect to inflammation are exactly the opposite of those of the above-mentioned inflammatory cytokines, have been found to be adsorbable and removable with an adsorbent comprising a compound having a log P value of at least 2.50, which is immobilized on a water-insoluble carrier.

The object of the present invention is to provide an adsorbent for efficiently adsorbing and removing at least one cytokine selected from the group consisting of IL-4, IL-10 and IL-13 in body fluids, a method for adsorbing and removing cytokine in a solution using the adsorbent and an adsorbe for the cytokine.

DISCLOSURE OF INVENTION

The present inventors have conducted intensive studies on adsorbents capable of efficiently adsorbing and removing at least one cytokine selected from the group consisting of IL-4, IL-10 and IL-13 in body fluids. As a result, the inventors have found that an adsorbent comprising a compound having a log P value of at least 2.50, which is immobilized on a water-insoluble carrier, can efficiently adsorb and remove at least one cytokine selected from the group consisting of IL-4, IL-10 and IL-13 in body fluids and the present invention was achieved.

Namely, the present invention relates to an adsorbent for at least one cytokine selected from the group consisting of IL-4, IL-10 and IL-13, comprising a compound having a log P value of at least 2.50 (P is a partition coefficient in an octanol-water system), which is immobilized on a water-insoluble carrier.

In a preferable embodiment, the water-insoluble carrier is a water-insoluble porous carrier.

Also, the present invention relates to a method for removing at least one cytokine selected from the group consisting of IL-4, IL-10 and IL-13 in a body fluid, which comprises contacting a body fluid with an adsorbent for at least one cytokine selected from the group consisting of IL-4, IL-10 and IL-13; the adsorbent comprising a compound having a log P value of at least 2.50 (P is a partition coefficient in an octanol-water system), which is immobilized on a water-insoluble carrier.

Furthermore, the present invention relates to an adsorber for removing at least one cytokine selected from the group consisting of IL-4, IL-10 and IL-13 comprising a container having an inlet and an outlet for a fluid and a means for preventing an adsorbent from flowing out of the container, which is filled with an adsorbent for at least one cytokine selected from the group consisting of IL-4, IL-10 and IL-13; the adsorbent comprising a compound having a log P value of at least 2.50 (P is a partition coefficient in an octanol-water system), which is immobilized on a water-insoluble carrier.

BRIEF EXPLANATION OF DRAWINGS

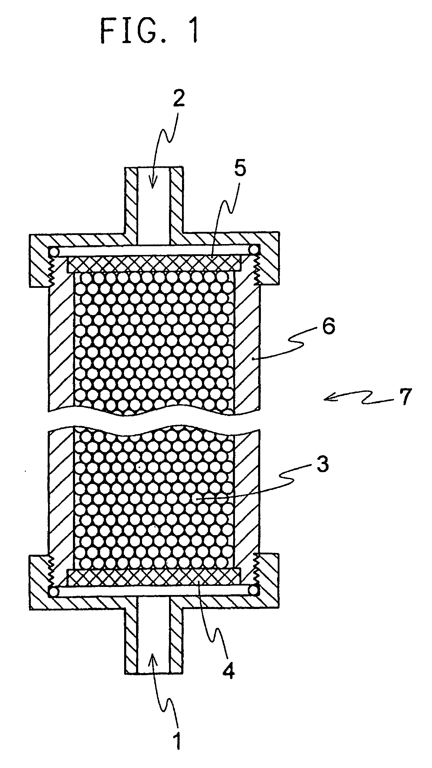

Fig. 1 is a schematic cross sectional view of one example of the adsorber for cytokine of the present invention. In Fig. 1, numeral 1 indicates an inlet for liquid, numeral 2 indicates an outlet for liquid, numeral 3 indicates the adsorbent for cytokine of the present invention, numerals 4 and 5 indicate filters through which liquid and components contained in the liquid can pass but the adsorbent for cytokine cannot pass, numeral 6 indicates a column and numeral 7 indicates an adsorber for cytokine.

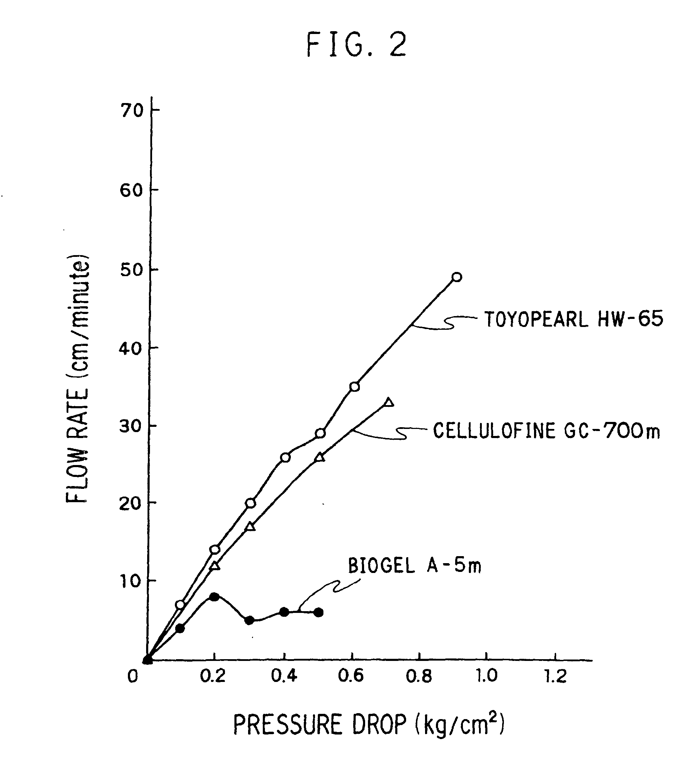

Figure 2 is a graph showing the results of examining the relationship between flow rate and pressure loss using three kinds of materials.

BEST MODE FOR CARRING OUT THE INVENTION

Herein, "IL-4" refers to glycoprotein having molecular weight of approximately 20 kDa.

"IL-10" refers to homodimer-type glycoprotein having molecular weight of approximately 35 to 40 kDa.

"IL-13" refers to glycoprotein having molecular weight of approximately 17 kDa.

"Body fluid" refers to blood, plasma, serum, ascites, lymph, synovia, fractions obtained therefrom and other liquid components obtained from a living body.

The adsorbent of the present invention comprises a compound having a log P value of at least 2.50, which is immobilized on a water-insoluble carrier. The log P value is a parameter that indicates the hydrophobicity of a compound. A typical partition coefficient P in an octanol-water system is calculated as described below. First, a compound is dissolved in octanol (or water) and an equal amount of water (or octanol) is added thereto. After shaking the mixture for 30 minutes in a Griffin flask shaker (made by Griffin & George Ltd.), the mixture is centrifuged for 1 to 2 hours at 2,000 rpm. The concentration of the compound in the octanol layer and the water layer is measured at room temperature under atmospheric pressure by various methods such as a spectroscopic method or GLC and the partition coefficient P is found from the following equation.

$$P = C_{oct}/C_w$$

$C_{oct}$: concentration of the compound in the octanol layer
$C_w$: concentration of the compound in the water layer

The adsorbent of the present invention comprises a compound having a log P value of at least 2.50 found in the above manner, which is immobilized on a water-insoluble carrier.

Until now, many researchers have measured the log P values of various compounds and the obtained values have been organized by C. Hansch et al. (cf. "PARTITION COEFFICIENTS AND THEIR USES"; Chemical Reviews, vol.71, page 525 (1971)).

With respect to compounds for which the value is unknown, the value ($\Sigma f$) calculated using hydrophobic fragmental constant f can be referred to, which is disclosed in "THE HYDROPHOBIC FRAGMENTAL CONSTANT", Elsevier Sci. Pub. Com., Amsterdam, (1977) by R.F. Rekker. The hydrophobic fragmental constant is a value indicating the hydrophobicity of various fragments determined by statistical analysis based on many found values of log P. The sum of the f values of respective fragments that constitute a compound has been reported to nearly correspond with the log P value of the compound.

In searching for compounds effective for adsorbing cytokine selected from IL-4, IL-10 and IL-13, a compound having a log P value of at least 2.50 is immobilized to a water-insoluble carrier. As a result, adsorption ability is found to be extremely high when hexadecylamine ($\Sigma f=7.22$) is immobilized on a water-insoluble carrier as the compound. From these results, adsorption of cytokine by the adsorbent of the present invention is considered to occur due to hydrophobic interaction between cytokine and an atomic group introduced onto the carrier by immobilizing a compound having a log P value of at least 2.50.

In the present invention, the compound that is immobilized to the water-insoluble carrier is not particularly limited as long as the compound has a log P value of at least 2.50. However, in the case of bonding the compound to the carrier by a chemical bonding method, part of the compound is often eliminated. When the eliminated group contributes largely to the hydrophobicity of the compound, that is when the hydrophobicity of the atomic group that is immobilized onto the carrier becomes smaller than $\Sigma f = 2.50$ due to elimination, the compound is not suitable as the compound used in the present invention, considering the purpose of the present invention. A typical example thereof is the case where isopentyl benzoate ($\Sigma f = 4.15$) is immobilized on a carrier having hydroxyl group by transesterification. In such a case, the atomic group that is actually immobilized onto the carrier is $C_6H_5$-CO- and the $\Sigma f$ value of the atomic group is at most 1. Whether such a compound is suitable as the compound used in the present invention can be determined by whether or not the log P value of a compound wherein the part of eliminated group is substituted with hydrogen is at least 2.50, or whether or not the hydorophobicity ($\Sigma f$) of the atomic group immobilized onto the carrier by elimination is at least 2.50.

Among compounds having a log P value of at least 2.50, preferable are compounds having a functional group that can be utilized for binding to the carrier such as unsaturated hydrocarbons, alcohols, amines, thiols, carboxylic acids and derivatives thereof, halides, aldehydes, hydrazides, isocyanates, compounds having an oxirane ring including glycidyl ether and halogenated silane. Typical examples of these compounds are amines such as n-heptylamine, n-octylamine, decylamine, dodecylamine, hexadecylamine, octadecylamine, 2-aminooctene, naphthylamine, phenyl-n-propylamine and diphenylmethylamine; alcohols such as n-heptyl alcohol, n-octyl alcohol, dodecyl alcohol, hexadecyl alcohol, 1-octene-3-ol, naphthol, diphenyl methanol and 4-phenyl-2-butanol, and glycidyl ethers of these alcohols; carboxylic acids such as n-octanoic acid, nonanoic acid, 2-nonenoic acid, decanoic acid, dodecanoic acid, stearic acid, arachidonic acid, oleic acid, diphenylacetic acid and phenylpropionic acid and derivatives thereof including acid halides, esters and amides; halides such as octyl chloride, octyl bromide, decyl chloride and dodecyl chloride; thiols such as octanethiol and dodecanethiol; halogenated silanes such as n-octyltrichlorosilane and octadecyltrichlorosilane; and aldehydes such as n-octylaldehyde, n-caprinaldehyde and dodecylaldehyde.

Besides these, compounds having a log P value of at least 2.50 among compounds wherein a hydrogen atom of the hydrocarbon part of the above compounds are substituted with a substituent containing a heteroatom such as halogen, nitrogen, oxygen and sulfur, or another alkyl group and compounds having a log P value of at least 2.50 that are shown in the tables of pages 555 to 613 of the above review by C. Hansch et al, "PARTITION COEFFICIENTS AND THEIR USES", Chemical Reviews, vol. 71, 525 (1971) can be used. However, the compounds are not limited thereto in the present invention.

These compounds may be used alone or any two or more kinds may be used together. These compounds can also be used together with a compound having a log P value of less than 2.50.

The water-insoluble carrier in the adsorbent of the present invention refers to a carrier that is solid under normal temperature and normal pressure and is insoluble in water. The water-insoluble carrier in the present invention may be in the form of particle, sheet, fiber or hollow fiber, but is not particularly limited. The size of the carrier is also not particularly limited.

For example, when the adsorbent of the present invention is in the form of particles, the average particle size is preferably at least 5 μm and at most 1000 μm. When the average particle size is less than 5 μm, in the case that cells are contained in body fluid, sufficient space through which the cells can pass may not be obtained. When the average particle size is more than 1000 μm, adsorption ability per volume may not sufficiently be obtained. From the viewpoint that cells can pass smoothly, the average particle size is more preferably at least 25 μm and at most 1000 μm. Particularly, from the viewpoint that cells can pass more easily and adsorption ability is obtained, the average particle size is preferably at least 40 μm and at most 600 μm. Specifically, when the body fluid is blood, the average particle size is preferably at least 200 μm and at most 1000 μm, from the viewpoint that blood cells can pass with ease, and more preferably at least 200 μm and at most 600 μm, from the viewpoint that adsorption ability is obtained. Also, the particle distribution is preferably narrow, for the reason that pressure loss is not increased.

When the adsorbent of the present invention is in the form of hollow fiber, the inner diameter is pref-

erably at least 1 μm and at most 500 μm. When the inner diameter is smaller than 1 μm, in the case that cells are contained in body fluid, the cells may not sufficiently pass through. When the inner diameter is larger than 500 μm, adsorption ability per volume tends to be insufficient. From the viewpoint that cells can pass through smoothly, the inner diameter is more preferably at least 2 μm and at most 500 μm. From the viewpoints that cells can pass through more easily and adsorption ability is obtained, the inner diameter is most preferably at least 5 μm and at most 200 μm.

Typical examples of the water-insoluble carrier used in the adsorbent of the present invention are inorganic carriers such as glass beads and silica gel; organic carriers comprising synthetic polymers such as crosslinked polyvinyl alcohol, cross-linked polyacrylate, cross-linked polyacrylamide and cross-linked polystyrene or polysaccharides such as crystalline cellulose, crosslinked cellulose, crosslinked agarose and crosslinked dextrin; and composite carriers obtained by combining the above such as organic-organic carriers and organic-inorganic carriers.

Of these, hydrophilic carriers are preferable, as non-specific adsorption is relatively small and adsorption selectivity for cytokine is favorable. The hydrophilic carrier refers to a carrier wherein the contact angle to water when the compound that constitutes the carrier is a flat plate is at most 60 degrees. Various methods for measuring contact angle to water are known and as shown in a book by Ikeda, (Experimental Chemical Selection: Colloid Chemistry, Chapter 4, Thermodynamics of Interface, pages 75-104 (1986) published by Shokabo), the method of measuring by placing a water droplet on a flat-plate compound is most common. Typical examples of a compound wherein the contact angle to water measured by this method is at most 60 degrees are carriers comprising cellulose, polyvinyl alcohol, hydrolyzed ethylene-vinyl acetate copolymer, polyacrylamide, polyacrylic acid, polymethacrylic acid, polymethyl methacrylate, polyethylene grafted with polyacrylic acid, polyethylene grafted with polyacrylamide and glass.

These water-insoluble carriers more preferably have a large number of micropores of a suitable size, that is have a porous structure. A carrier having a porous structure includes a carrier having space (macropores) that is formed by agglomeration of microspherical particles when the base polymer matrix forms one spherical particle by agglomeration of microspherical particles, a carrier having micropores between the agglomerate of nuclei in one microparticle that constitutes the base polymer matrix and a carrier having micropores when a copolymer having a three-dimensional structure (polymer network) is swelled with an organic solvent having affinity therewith.

From the viewpoint of adsorption ability per unit volume of the adsorbent, the water-insoluble carrier having a porous structure is preferably porous overall rather than porous only on the surface. Also, the pore volume and specific surface area are preferably large, as long as adsorption ability is not lost.

An example of a carrier satisfying these preferable conditions is porous cellulose gel. Porous cellulose gel has the following advantages:

(1) porous cellulose gel is not destroyed or does not generate fine powder by operations such as stirring because the gel has relatively high mechanical strength and is tough, can be flowed at a high flow rate as the gel dos not consolidate when packed in a column even when body fluid is poured at a high flow rate and has a micropore structure that is unsusceptible to change by high pressure steam sterilization;

(2) porous cellulose gel is hydrophilic as the gel is constituted by cellulose, contains a large number of hydroxyl groups that can be used for bonding ligand and has low non-specific adsorption;

(3) adsorption volume that is not inferior to soft get can be obtained as porous cellulose gel has relatively high strength even when pore volume is large; and

(4) porous cellulose gel is safer than synthetic polymer gel. Consequently, porous cellulose gel is one of the most suitable carriers for the present invention. However, the carrier of the present invention is not limited thereto. The above carriers may be used alone or any two or more kinds can be used together.

These water-insoluble carriers having a porous structure preferably have the feature that the substance to be adsorbed can enter the micropores in a relatively large probability but entrance of other proteins is prevented as much as possible. The substances to be adsorbed by the adsorbent of the present invention are IL-4, which is a protein having a molecular weight of approximately 20 kDa, IL-10, which is a protein having a molecular weight of approximately 35 to 40 kDa, and IL-13, which is a protein having a molecular weight of approximately 17 kDa. In order to efficiently adsorb these proteins, the cytokine must be able to enter into the micropores in a relatively large probability, but entrance of other proteins is prevented as much as possible. As the standard for molecular weight of a substance that can enter into the micropores, exclusion limit molecular weight is generally used. The exclusion limit molecular weight refers to the molecular weight of the molecule having the smallest molecular weight, among molecules that cannot enter into (are excluded from) the micropores in gel permeation chromatography, as described in books (for example, Hiroyuki Hatano and Toshihiko Hanai, "Experimental High Performance Liquid Chromatography", Kagaku Dojin). In general, the exclusion limit molecular weight of globular proteins, dextrans and polyethlene glycol has been eagerly studied. With respect to the exclusion limit of the carrier used in the present invention, the values obtained using globular protein are suitably used.

As a result of studying carriers of various exclusion limit molecular weight, the range of exclusion limit molecular weight of globular protein suitable for adsorbing the cytokines has been found to be at least 5,000 and at most 600,000. When a carrier having an exclusion limit molecular weight of globular protein of less than 5,000 is used, the amount of adsorbed cytokines is small and practicability decreases. When a carrier having an exclusion limit molecular weight of globular protein of more than 600,000 is used, the amount of protein (mainly albumin) other than the cytokines that is adsorbed increases and practicability decreases from the viewpoint of selectivity. Accordingly, the exclusion limit molecular weight of globular protein of the carrier used in the present invention is preferably at least 5,000 and at most 600,000. From the viewpoint that adsorption ability and selectivity can be obtained more effectively, the exclusion limit molecular weight is more preferably at least 6,000 and at most 400,000, most preferably at least 10,000 and at most 300,000.

The carrier preferably has a functional group that can be used in immobilization reaction of a ligand. Typical examples of the functional group are hydroxyl group, amino group, aldehyde group, carboxyl group, thiol group, silanol group, amide group, epoxy group, halogen group, succinylimide group and acid anhydride group, but not limited thereto.

Both a hard carrier and a soft carrier can be used as the carrier of the present invention. When using the adsorbent for extracorporeal circulation, clogging must not occur when the adsorbent is packed into the column and fluid is passed through and therefore, sufficient mechanical strength is required. Consequently, the carrier used in the present invention is preferably a hard carrier. Herein, hard carrier refers to in the case of a granular gel, a carrier wherein the relationship between pressure loss $\Delta P$ and flow rate, when the gel is evenly packed in a cylindrical column and an aqueous fluid is passed through, is a linear relation up to 0.3 kg/cm$^2$, as described below in Reference Example.

The adsorbent of the present invention is obtained by immobilizing a compound having a log P value of at least 2.50 on a water-insoluble porous carrier. Various known methods for immobilization can be used without any particular restriction. However, when the adsorbent of the present invention is used for an extracorporeal circulation treatment, from the viewpoint of safety, suppressing desorption and elution of ligands as much as possible in sterilization or treatment is important. Therefore, immobilization is preferably conducted by covalent bonding.

The immobilized amount of the compound having a log P value of at least 2.50 in the absorbent of the present invention is preferably at least 1 µmol/g (wet weight) and at most 5000 µmol/g (wet weight). When the immobilized amount is less than 1 µmol/g (wet weight), adsorption of cytokine tends to be insufficient. When the immobilized amount is more than 5000 µmol/g (wet weight), in that case that the fluid is blood, adhesion of platelets tends to occur. From the viewpoints of effective adsorption ability and little adhesion of platelets, the immobilized amount of the compound is more preferably at least 5 µmol/g (wet weight) and at most 3000 µmol/g (wet weight).

There are various methods for adsorbing and removing the cytokine from body fluid using the adsorbent of the present invention. The most simple method is the method of retrieving body fluid and storing in a bag, mixing the adsorbent thereto to adsorb and remove the cytokine and then filtering the adsorbent to obtain the body fluid from which the cytokine has been removed. Another method is the method of filling a container having an inlet and an outlet for body fluid, wherein a filter that passes body fluid but not the adsorbent is installed to the outlet, with the adsorbent and then pouring body fluid. Both methods can be used, but the latter method is suited for the adsorbent of the present invention, as operation is simple and the cytokine can be removed efficiently by on-line system from body fluid, especially blood, of a patient by incorporating the method into an extracorporeal circulation circuit.

In the extracorporeal circulation circuit, the adsorbent of the present invention can be used alone or together with other extracorporeal circulation therapy systems. An example of using together with another is artificial dialysis circuit and the adsorbent can be together with dialysis therapy.

Below, the adsorber for cytokine of the present invention using the above cytokine adsorbent is explained based on Fig. 1, which is schematic cross sectional view of one example of the adsorber. In Fig. 1, numeral 1 indicates an inlet for liquid, numeral 2 indicates an outlet for liquid, numeral 3 indicates the adsorbent for cytokine of the present invention, numerals 4 and 5 indicate filters through which liquid and components contained in the liquid can pass but the adsorbent for cytokine cannot pass, numeral 6 indicates a column and numeral 7 indicates an adsorber for cytokine. The adsorber for cytokine of the present invention is not limited to this example and any device can be used as long as the device has an inlet and an outlet for fluid and the adsorbent is packed in a container equipped with a means to prevent the adsorbent for cytokine from flowing out of the container.

Examples of the means for preventing the adsorbent from flowing out are, for instance, filters such as mesh, nonwoven fabric and cotton plug. The shape, material and size of the container are not particularly limited, but the shape of the container is preferably cylindrical. The material of the container is preferably a material having sterilization resistance and specific examples are glass coated with silicone, polypropylene, polyvinyl chloride, polycarbonate, polysulfone and polymethylpentene. The capacity of the container is preferably at least 50 ml and at most 1500 ml, and the diameter is preferably at least 2 cm and at most 20 cm. When the capacity of the container is less than 50 ml, the adsorbed amount is insufficient, whereas when the capacity of the container is more than 1500 ml, the extracorporeally circulated amount becomes large, thus being unfavorable. When the diameter of the container is less than 2 cm, the pressure loss becomes large as the linear velocity is high, thus being unfavorable. When the diameter of the container is more than 20 cm, coagulation may occur as handling is difficult and linear velocity is low, thus being unfavorable. From the viewpoints of an effective adsorption amount and excellent safety, the container more preferably has capacity of at least 100 ml and at most 800 ml and diameter of at least 3 cm and at most 15 cm, particularly preferably capacity of at least 150 ml and at most 400 ml and diameter of at least 4 cm and at most 10 cm.

Hereinafter, the present invention is described in more detail by means of Examples, but the present invention is not limited thereto.

REFERENCE EXAMPLE

A cylindrical glass column (inner diameter 9 mm, length 150 mm) equipped with filters having a pore size of 15 μm at both ends was uniformly charged with each of agarose material (Biogel A-5m available from Bio-Rad Laboratories, particle size 50 to 100 mesh), vinyl polymer material (TOYOPEARL HW-65 available from TOSOH Corporation, particle size 50 to 100 μm) and cellulose material (CELLULOFINE GC-700m available from Chisso Corporation, particle size 45 to 105 μm). Then, water was passed through by a peristatic pump. The relationship between flow rate and pressure loss ΔP was found. The results are shown in Fig. 2.

As shown in Fig. 2, the flow rate of TOYOPEARL HW-65 and CELLULOFINE GC-700m increase almost in proportion to increase in pressure. On the other hand, Biogel A-5m consolidates and the flow rate does not increase even when the pressure is increased. In the present invention, a material wherein the relationship between pressure loss ΔP and flow rate is a linear relation up to 0.3 kg/cm$^2$, as in the case of the former, is referred to as a hard material.

EXAMPLE 1

Water was added to 170 ml of porous cellulose gel CELLULOFINE GC-200m (available from Chisso Corporation, particle size: 45 to 105 μm, exclusion limit molecular weight for globular protein: 140,000) so that the total amount became 340 ml. Then, 90 ml of a 2M aqueous solution of sodium hydroxide was added thereto, and the temperature was adjusted to 40°C. To the mixture was added 31 ml of epichlorohydrin and reaction was carried out while stirring at 40°C for 2 hours. After the reaction was completed, the gel was thoroughly washed with water to obtain an epoxidized gel.

To 10 ml of the epoxidized gel was added 200 mg of n-hexadecylamine (Σf = 7.22) and the mixture was left still to react in ethanol at 45°C for 6 days to immobilize. After the reaction was completed, the gel was thoroughly washed with ethanol and then with water to obtain n-hexadecylamine-immobilized gel (immobilized amount of hexadecylamine: 45 μmol/g (wet weight)).

To 0.5 ml of the immobilized gel was added 3 ml of cytokine-containing serum of a healthy volunteer prepared by adding human gene recombinant IL-4, IL-10 and IL-13 (all available from R & D systems) to serum of a healthy volunteer (available from Dainippon Pharmaceutical Co., Ltd.). The mixture was incubated at 37°C for 2 hours. After incubation, the concentration of each cytokine in the supernatant was measured using a human IL-4 measurement kit, a human IL-10 measurement kit and a human IL-13 measurement kit (all available from Biosource International Corporation). The results are shown in Table 1.

EXAMPLE 2

n-Octylamine-immobilized gel (immobilized amount of n-octylamine: 43 μmol/g (wet weight)) was obtained in the same manner as in Example 1, except that n-octylamine (log P=2.90) was used instead of n-hexadecylamine and 50 (v/v) % aqueous solution of ethanol was used as the medium for immobilization instead of ethanol. Adsorption test was carried out in the same manner as in Example 1 using this immobilized gel and the concentration of each cytokine was measured. The results are shown in Table 1.

COMPARATIVE EXAMPLE 1

n-Hexylamine-immobilized gel (immobilized amount of n-hexylamine: 46 μmol/g (wet weight)) was obtained in the same manner as in Example 2 except that n-hexylamine (log P=2.06) was used instead of n-octylamine. Adsorption test was carried out in the same manner as in Example 1 using this immobilized gel and the concentration of each cytokine was measured. The results are shown in Table 1.

COMPARATIVE EXAMPLE 2

n-Butylamine-immobilized gel (immobilized amount of n-butylamine: 48 μmol/g (wet weight)) was obtained in the same manner as in Example 2 except that n-butylamine (log P=0.97) was used instead of n-octylamine. Adsorption test was carried out in the same manner as in Example 1 using this immobilized gel and the concentration of each cytokine was measured. The results are shown in Table 1.

COMPARATIVE EXAMPLE 3

Adsorption test was carried out in the same manner as in Example 1 using CELLULOFINE GC-200m, on which a ligand was not immobilized, and the concentration of each cytokine was measured. The results are shown in Table 1.

TABLE 1

| | Cytokine Concentration (pg/ml) | | |
|---|---|---|---|
| | IL-4 | IL-10 | IL-13 |
| Ex. 1 | 9 | 65 | 150 |
| Ex. 2 | 15 | 80 | 265 |
| Com. Ex. 1 | 240 | 385 | 2000 |
| Com. Ex. 2 | 240 | 390 | 2100 |
| Com. Ex. 3 | 250 | 400 | 2300 |

As can be seen from Table 1, the concentration of each cytokine in the supernatant is significantly decreased in Examples 1 and 2 compared to Comparative Examples 1, 2 and 3.

EXAMPLE 3

Cellulose acetate was dissolved in a mixed solvent of dimethylsulfoxide and propylene glycol. The solution was dropped by the method described in JP-A-63-117039 (vibration method) and coagulated to obtain spherical hydrogel particles of cellulose acetate. The hydrogel particles were mixed with an aqueous solution of sodium hydroxide and hydrolysis reaction was conducted to obtain hydrogel particles of cellulose (average particle size: 460 μm, exclusion limit moleuclar weight for globular protein: 50,000). The hydrogel particles were reacted with epichlorohydrin in the same manner as in Example 1 and n-hexadecylamine was immobilized onto the particles to obtain n-hexadecylamine-immobilized particles (immobilized amount of n-hexadecylamine: 35 μmol/g (wet weight)). Adsorption test was carried out in the same manner as in Example 1 using the immobilized particles and the concentration of each cytokine was measured. The results are shown in Table 2.

EXAMPLE 4

n-Octylamine-immobilized particles (immobilized amount of n-octylamine: 33 μmol/g (wet weight)) were obtained in the same manner as in Example 3 except that n-octylamine was used instead of n-hexadecylamine and that 50 (v/v) % aqueous solution of ethanol was used as the medium for immobilization reaction instead of ethanol. Adsorption test was carried out in the same manner as described in Example 1 using the immobilized particles and the concentration of each cytokine was measured. The results are shown in Table 2.

COMPARATIVE EXAMPLE 4

Adsorption test was carried out in the same manner as in Example 1 using the hydrogel particles of cellulose on which a ligand was not immobilized prepared in Example 3 and the concentration of each cytokine was measured. The results are shown in Table 2.

TABLE 2

| | Cytokine Concentration (pg/ml) | | |
|---|---|---|---|
| | IL-4 | IL-10 | IL-13 |
| Ex. 3 | 10 | 70 | 170 |
| Ex. 4 | 20 | 80 | 270 |
| Com. Ex. 4 | 260 | 390 | 2300 |

As can be seen from Table 2, the concentration of each cytokine in the supernatant is significantly decreased in Examples 3 and 4 compared to Comparative Example 4.

INDUSTRIAL APPLICABILITY

By using the adsorbent obtained by immobilizing a compound having a log P value of at least 2.50 onto a water-insoluble carrier according to the method of the present invention, at least one cytokine selected from the group consisting of IL-4, IL-10 and IL-13 can be efficiently adsorbed and removed.

**Claims**

1. An adsorbent for adsorbing at least one cytokine selected from the group consisting of interleukin-4, interleukin-10 and interleukin-13,
comprising a compound having a log P value of at least 2.50 (P is a partition coefficient in an octanol-water system), which is immobilized on a water-insoluble carrier.

2. The adsorbent of Claim 1, wherein said water-insoluble carrier is a water-insoluble porous carrier.

3. A method for removing at least one cytokine selected from the group consisting of interleukin-4, interleukin-10 and interleukin-13 in a body fluid, which comprises contacting a body fluid with an adsorbent for adsorbing at least one cytokine selected from the consisting of interleukin-4, interleukin-10 and interleulkin-13;
said adsorbent comprising a compound having a log P value of at least 2.50 (P is a partition coefficient in an octanol-water system), which is immobilized on a water-insoluble carrier.

4. An adsorber for adsorbing at least one cytokine selected from the group consisting of interleukin-4, interleukin-10 and interleulkin-13, comprising a container having an inlet and an outlet for a body fluid and

a means for preventing an adsorbent from flowing out of said container, which is filled with an adsorbent for adsorbing at least one cytokine selected from the consisting of interleukin-4, interleukin-10 and interleukin-13;

said adsorbent comprising a compound having a log P value of at least 2.50 (P is a partition coefficient in an octanol-water system), which is immobilized on a water-insoluble carrier.

5. Use of an adsorbent comprising a compound having a log P value of at least 2.50 (P is a partition coefficient in an octanol-water system), which is immobilized on a water-insoluble carrier, for adsorbing and removing from a body liquid at least one cytokine selected from the group consisting of interleukin-4, interleukin-10 and interleukin-13.

# FIG. 1

# FIG. 2

## INTERNATIONAL SEARCH REPORT

| | International application No. |
|---|---|
| | PCT/JP02/13207 |

A. CLASSIFICATION OF SUBJECT MATTER
Int.Cl⁷ A61M1/36, B01J20/26

According to International Patent Classification (IPC) or to both national classification and IPC

B. FIELDS SEARCHED

Minimum documentation searched (classification system followed by classification symbols)
Int.Cl⁷ A61M1/36, B01J20/26

Documentation searched other than minimum documentation to the extent that such documents are included in the fields searched
Jitsuyo Shinan Koho          1922-1996   Toroku Jitsuyo Shinan Koho   1994-2003
Kokai Jitsuyo Shinan Koho    1971-2003   Jitsuyo Shinan Toroku Koho   1996-2003

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y | JP 9-501066 A (Rhône Poulenc Rorer Pharmaceuticals, Inc.), 04 February, 1997 (04.02.97), Full text; Figs. 1 to 10 & WO 94/21124 A1 | 1,2,4,5 |
| Y | WO 90/09396 A1 (Kuraray Co., Ltd.), 23 August, 1990 (23.08.90), Full text & US 5171837 A | 1,2,4,5 |
| A | JP 8-257398 A (Kaneka Corp.), 08 October, 1996 (08.10.96), Full text; Figs. 1, 2 (Family: none) | 1,2,4,5 |

| ☒ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 26 March, 2003 (26.03.03) | 15 April, 2003 (15.04.03) |

| Name and mailing address of the ISA/ | Authorized officer |
|---|---|
| Japanese Patent Office | |
| Facsimile No. | Telephone No. |

Form PCT/ISA/210 (second sheet) (July 1998)

## INTERNATIONAL SEARCH REPORT

International application No.

PCT/JP02/13207

C (Continuation). DOCUMENTS CONSIDERED TO BE RELEVANT

| Category* | Citation of document, with indication, where appropriate, of the relevant passages | Relevant to claim No. |
|---|---|---|
| A | JP 8-257115 A  (Kaneka Corp.),<br>08 October, 1996 (08.10.96),<br>Full text; Figs. 1, 2<br>(Family: none) | 1,2,4,5 |

Form PCT/ISA/210 (continuation of second sheet) (July 1998)

**INTERNATIONAL SEARCH REPORT**

International application No.

PCT/JP02/13207

**Box I  Observations where certain claims were found unsearchable (Continuation of item 2 of first sheet)**

This international search report has not been established in respect of certain claims under Article 17(2)(a) for the following reasons:

1. ☒ Claims Nos.: 3
   because they relate to subject matter not required to be searched by this Authority, namely:
   Claim 3 pertains to methods for treatment of the human body by surgery or therapy and thus relates to a subject matter which this International Searching Authority is not required, under the provisions of Article 17(2)(a)(i) of the PCT and Rule 39.1(iv) of the Regulations under the PCT, to search.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box II  Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**  ☐ The additional search fees were accompanied by the applicant's protest.
                        ☐ No protest accompanied the payment of additional search fees.

Form PCT/ISA/210 (continuation of first sheet (1)) (July 1998)